(19) Europäisches Patentamt
European Patent Office
Office européen des brevets

(11) Veröffentlichungsnummer: **0 153 988**
**B1**

(12) ## EUROPÄISCHE PATENTSCHRIFT

(45) Veröffentlichungstag der Patentschrift:
06.06.88

(51) Int. Cl.⁴: **A 61 M 16/01**

(21) Anmeldenummer: **84110542.2**

(22) Anmeldetag: **05.09.84**

(54) Vorrichtung zur Beimischung flüssiger Narkosemittel in das dem Patienten zuzuführende Atemgas.

(30) Priorität: **20.01.84 DE 3401923**

(43) Veröffentlichungstag der Anmeldung:
**11.09.85 Patentblatt 85/37**

(45) Bekanntmachung des Hinweises auf die Patenterteilung:
**06.06.88 Patentblatt 88/23**

(84) Benannte Vertragsstaaten:
**CH DE FR GB IT LI NL SE**

(56) Entgegenhaltungen:
**DD-A-200 498**
**DE-A-3 116 951**
**GB-A-1 316 095**
**US-A-3 465 753**

(73) Patentinhaber: **Drägerwerk Aktiengesellschaft, Moislinger Allee 53- 55, D-2400 Lübeck 1 (DE)**

(72) Erfinder: **Ryschka, Martin, Dr. Dipl.- Phys., Morierstrasse 10b, D-2406 Stockelsdorf (DE)**
Erfinder: **Falb, Wolfgang, Dipl.- Ing., Ruschweg 11, D-2401 Krummesse (DE)**
Erfinder: **Büttner, Peter, Dipl.- Ing., Kirchenstrasse 5, D-2407 Bad Schwartau (DE)**
Erfinder: **Wallroth, Carl Friedrich, Dr. Dipl.- Ing., Stresemannstrasse 1, D-2400 Lübeck (DE)**

## Beschreibung

Die Erfindung betrifft eine Vorrichtung zur Beimischung flüssiger Narkosemittel in das einem Patienten zuzuführende Atemgas, gemäß dem ersten Teil des Anspruchs 1.

Die Geräte der Narkosetechnik sollten die Möglichkeit bieten, durch Umschaltung nach Bedarf verschiedene Narkosemittel einzusetzen. Sie sollten mit gleicher Exaktheit sowohl in den Frischgasstrom als auch direkt in das Patientensystem eingeleitet werden können.

Eine aus der DE-A-31 16 951 bekannte Vorrichtung zur Beimischung flüssiger Narkosemittel in das dem Patienten zuzuführende Atemgas enthält eine aufheizbare Wirbelkammer, in die hinein das Narkosemittel durch eine Pumpe aus dem Narkosemittelbehälter heraus zugeführt wird. Es tritt dabei mit dem Atemgasstrom tangential in die Wirbelkammer ein und wird dann in der sich ausbildenden zyklonartigen Strömung verdunstet. Eine Heizung der Wirbelkammer, gesteuert durch einen Temperaturfühler, deckt die Verdunstungswärme. Das Atemgas strömt durch einen Auslaß, in dem seine Temperatur überprüft wird, wieder aus. Das mit dem Narkosemittel angereicherte Atemgas wird dann dem Patienten zugeführt. Eine mit Fühlern verbundene Steuer- und Auswerteschaltung führt die Messung durch und löst bei Abweichungen von Sollwerten Alarme oder Regelungen aus.

In der US-A-3 465 753 ist in einem geschlossenen Atemkreislauf ein Narkosemittelverdunster parallel zu einem Narkosegerät geschaltet. In der zu dem Verdampfer führenden Atemgasleitung befindet sich eine Überwachungseinheit, die den Gehalt an Narkosegas überprüft. Wenn der Narkosegehalt geringer ist als der vorher eingestellte gewünschte Gehalt, wird ein strömungsaufwärts des Verdunsters angeordneter Umschalter derart betätigt, daß über eine Pumpe ein Teil des Atemgases über den Verdunster geleitet wird, so daß dieses solange mit Narkosegas angereichert wird, bis der geforderte Narkosegehalt im Atemkreislauf erreicht ist. Daraufhin wird der Umschalter wieder in seine Ausgangslage gebracht, wodurch der Verdunster strömungsmäßig aus dem Atemkreislauf wieder herausgenommen wird.

Aufgabe der Erfindung ist, eine Vorrichtung zur Beimischung flüssiger Narkosemittel in das dem Patienten zuzuführende Atemgas zu schaffen, in der verschiedene Narkosemittel umschaltbar zur Verfügung stehen, die dann nach Verdampfung in das Atemgassystem, sowohl in das Frischgas als auch direkt in das Patientensystem eingeleitet werden können, wobei die Vorrichtung durch den Benutzer sicher zu übersehen und einfach zu schalten sein soll.

Die Lösung der Aufgabe erfolgt bei einer Vorrichtung zur Beimischung flüssiger Narkosemittel in das einem Patienten zuzuführende Atemgas nach dem ersten Teil des Anspruchs 1 mit den technischen Merkmalen seines kennzeichnenden Teils.

Die mit der Erfindung erzielten Vorteile bestehen insbesondere darin, daß in Anwendung an einem geschlossenen Atemsystem, wo der Narkosemittelverdampfer benötigt wird, um ausreichende Narkosemittelmengen in das Atemgassystem hineinzubringen, für die Anwendung an einem offenen Atemsystem, in dem große Narkosemittelmengen benötigt werden, die gleichmäßig über ein Puffervolumen bereitgestellt werden müssen, die Wirbelkammer zur Verfügung steht.

Ein Ausführungsbeispiel der Erfindung ist in der Zeichnung dargestellt und wird im folgenden beschrieben. Es zeigen

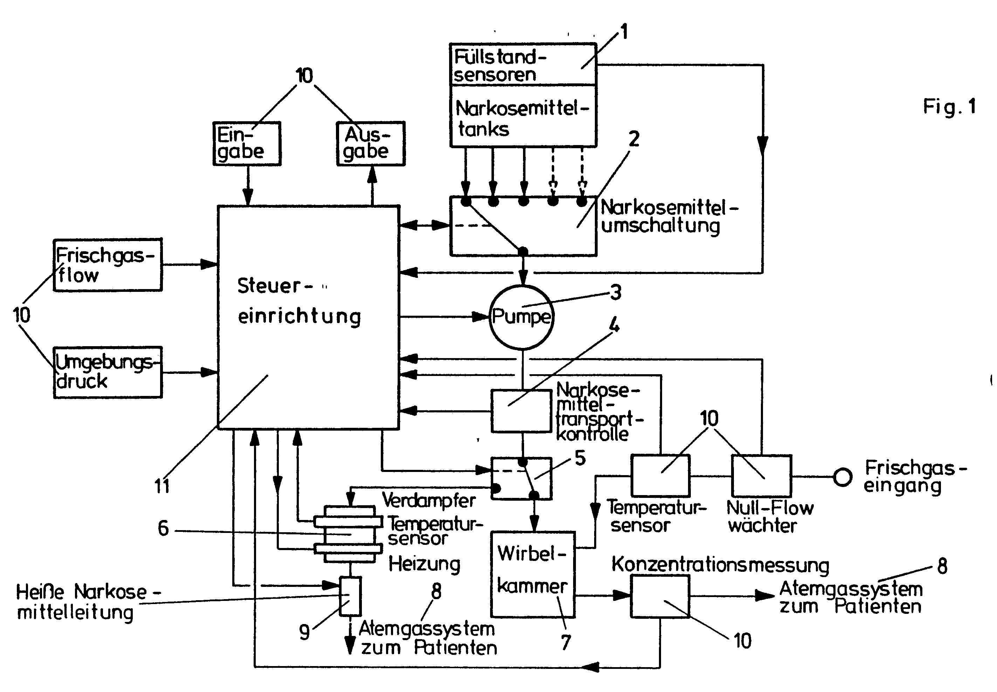

Fig. 1 das Schaltschema der Vorrichtung
Fig. 2 die Narkosemittelumschaltung
Fig. 3 Überwachung des Motors der Pumpe

Die Vorrichtung zur Beimischung flüssiger Narkosemittel in das dem Patienten zuzuführende Atemgas enthält in der Reihenfolge ihrer Durchströmung durch die Narkosemittel die folgenden Bauelemente:

einen oder mehrere Narkosemitteltanks 1 mit den notwendigen Füllstandsanzeigen und Dosierungseinrichtungen

eine Narkosemittelumschaltung 2 zur wahlweisen Umschaltung auf die verschiedenen Narkosemittel

eine Pumpe 3

eine Narkosemitteltransportkontrolle 4

einen Umschalter 5 zur Umschaltung des Narkosemittelstroms wahlweise auf einen Narkosemittelverdampfer 6 und eine Wirbelkammer 7.

Das verdampfte bzw. verdunstete Narkosemittel wird aus dem Narkosemittelverdampfer 6 bzw. der Wirbelkammer 7 in das Atemgassystem 8 (das Patientensystem oder das Frischgas) zum Patienten eingeleitet. Die Verbindung vom Narkosemittelverdampfer 6 zum Atemgassystem 8 erfolgt über eine mit einer Heizung versehene Rohrleitung 9. Meßeinrichtungen 10 überwachen die Daten und leiten sie zu einer Steuereinrichtung 11, von der sie ausgewertet zur Regelung an die Geräteelemente gegeben werden.

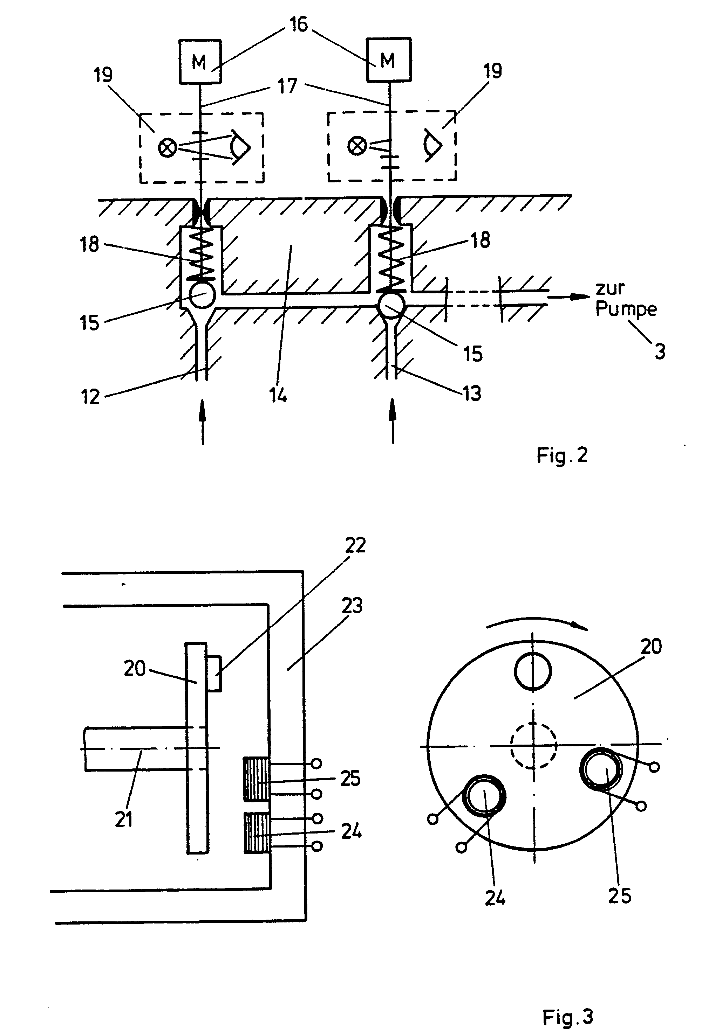

Von jedem Narkosemitteltank 1 führt je eine Rohrleitung 12, 13 zu der Narkosemittelumschaltung 2 (Fig. 2).

Dabei enden die Rohrleitungen 12, 13 in einem Gehäuse 14 in Kugelventilen 15. Die Kugelventile 15 werden über Elektromagnete 16 durch Anheben eines Stößels 17 geöffnet bzw. nach dem Lösen durch eine Feder 18 wieder geschloßen. Lichtschranken 19 an den Stößeln 17 zeigen den Schaltstand und steuern die Kugelventile 15.

Die Überwachung des Motors der Pumpe 3 (Fig. 3) erfolgt mittels einer Scheibe 20, die an der Rotorachse 21 des Motors der Kolbenpumpe befestigt ist und die einen Dauermagneten 22 trägt. Der Dauermagnet 22 wird im Umlauf an zwei in Abstand voneinander am Motorgehäuse 23 befestigten Induktionsspulen 24, 25 vorbeigeführt. Die induzierten Impulse geben Aufschluß darüber, ob der Rotor rotiert, in welcher Richtung und mit welcher Geschwindigkeit. Treten die Impulse außerhalb der vorgeschriebenen Zeitfenster auf, so wird eine Warnung erzeugt und/oder die Pumpe abgeschaltet.

## Patentansprüche

1. Vorrichtung zur Beimischung flüssiger Narkosemittel in das einem Patienten zuzuführende Atemgas, wobei das Narkosemittel durch eine steuerbare Dosierpumpe (3) über eine Narkosemittelzuführung in eine Wirbelkammer (7) gefördert wird, die von einem Frischgas als Trägergas durchströmbar ist, welches nach Zumischung und Verdunstung des Narkosemittels als Atemgas einem zum Patienten führenden Leitungssystem (8) zuführbar ist, dadurch gekennzeichnet, daß in der Narkosemittelzuführung strömungsaufwärts zur Wirbelkammer (7) ein Umschalter (5) vorgesehen ist, durch den das flüssige Narkosemittel unter Umgehung der Trägergasleitung und der Wirbelkammer (7) zu einem Verdampfer (6) umlenkbar ist, von welchem aus das verdampfte Narkosemittel über eine beheizbare Narkosemitteldampf-Leitung (9) in ein das Atemgas zum Patienten führendes, geschlossenes Leitungssystem (8) einspeisbar ist.

2. Vorrichtung zur Beimischung flüssiger Narkosemittel nach Anspruch 1, dadurch gekennzeichnet, daß der Dosierpumpe (3) eine Vielzahl von Narkosemitteltanks (1), die einzeln über eine Narkosemittelumschaltung (2) in die Narkosemittelzuführung einschaltbar sind, vorgeschaltet ist.

3. Vorrichtung zur Beimischung flüssiger Narkosemittel nach einem der Ansprüche 1 oder 2, dadurch gekennzeichnet, daß die Narkosemittelumschaltung (2) Kugelventile (15) enthält, deren über Elektromagnet (16) betätigte Stößel (17) in ihrer Stellung durch Lichtschranken (19) gesteuert überwacht werden.

4. Vorrichtung zur Beimischung flüssiger Narkosemittel nach einem der Ansprüche 1 bis 3, dadurch gekennzeichnet, daß die motorgetriebene Pumpe (3) an der Rotorachse (21) eine Scheibe besitzt, die einen Dauermagneten (22) trägt, der im Umlauf an zwei in Abständen voneinander am Motorgehäuse (23) befestigten Induktionsspulen (24, 25) vorbeigeführt wird.

## Claims

1. Apparatus for admixing a liquid anaesthetic into the respiratory gas to be supplied to a patient, whereby the anaesthetic is conveyed through a controllable dosing pump (3), by way of an anaesthetic supply line, into a centrifugal chamber (7) which is able to have fresh gas as carrier gas flowing through it, said gas, after admixture and evaporation of the anaesthetic, being able to be supplied as respiratory gas to a supply system (8) leading to the patient, characterised in that there is provided in the anaesthetic supply line, upstream of the centrifugal chamber (7), a change-over switch (5) by means of which the liquid anaesthetic can be diverted to an evaporator, by-passing the carrier gas supply line and the centrifugal chamber (7), the vaporised anaesthetic being able to be fed from the evaporator, by way of a heatable anaesthetic vapour supply line, into a closed supply system (8) leading the respiratory gas to the patient.

2. Apparatus for admixing a liquid anaesthetic according to claim 1, characterised in that a plurality of anaesthetic tanks (1), which are able to be connected individually into the anaesthetic supply by way of an anaesthetic change-over (2), are provided upstream of the dosing pump (3).

3. Apparatus for admixing a liquid anaesthetic according to one of claims 1 or 2, characterised in that the anaesthetic change-over (2) contains ball valves (15), the tappets (17) of which, activated by way of an electromagnet (16), are monitored in their position in a controlled manner by using light barriers (19).

4. Apparatus for admixing a liquid anaesthetic according to one of claims 1 to 3, characterised in that the motor-driven pump (3) possesses a disc on the rotor axis (21) which carries a permanent magnet (22) which, in the cycle, is led past two induction coils (24, 25) fixed at a distance from each other on the motor housing (23).

## Revendications

1. Dispositif pour l'incorporation d'un produit anesthésique liquide au gaz respiratoire destiné à un patient, le produit anesthésique étant, par une pompe de dosage asservissable (3) et par l'intermédiaire d'une conduite d'alimentation en produit anesthésique, refoulé dans une chambre de turbulence (7) qui peut être traversée par un gaz frais jouant le rôle de gaz porteur qui, à la suite de l'incorporation et de la vaporisation du produit anesthésique, peut être fourni comme gaz respiratoire à un système de tuyauterie (8) menant au patient, caractérisé en ce qu'un commutateur (5) est prévu en amont (dans le sens d'écoulement) de la chambre de turbulence (7) dans la conduite d'alimentation en produit anesthésique, commutateur par l'intermédiaire duquel le produit anesthésique liquide peut, en contournant la conduite de gaz porteur et la chambre de turbulence (7), être dévié vers un vaporisateur (6) à partir duquel le produit anesthésique vaporisé peut, en passant par une conduite de vapeur de produit anesthésique, apte à être chauffée, (9), être introduit dans un système de tuyauterie fermé (8) amenant le gaz respiratoire au patient.

2. Dispositif pour l'incorporation de produit anesthésique liquide selon la revendication 1, caractérisé en ce que plusieurs reservoirs (1) de produit anesthésique, qui peuvent être reliés individuellement à la conduite d'alimentation en produit anesthésique par l'intermédiaire d'un dispositif de commutation de produit anesthésique (2), sont montés en amont de la pompe de dosage (3).

3. Dispositif pour l'incorporation de produit anesthésique liquide selon la revendication 1 ou 2, caractérisé en ce que le dispositif de commutation de produit anesthésique (2) contient des soupapes à billes (15) dont les poussoirs (17) actionnés par des électro-aimants (16) sont surveillés et commandés quant à leur position par des barrages photoélectriques (19).

4. Dispositif pour l'incorporation de produit anesthésique liquide selon l'une des revendications 1 à 3, caractérisé en ce que la pompe motorisée (3) possède sur l'axe de rotor (21) un disque qui porte un aimant permanent (22) qui lors de la rotation passe devant deux bobines d'induction (24, 25) fixées à distance l'une de l'autre sur le bâti (23) du moteur.

Fig. 1

0 153 988

Fig. 2

Fig. 3

3